# EUROPÄISCHE PATENTSCHRIFT

(11) **EP 2 392 842 B1**
(45) Veröffentlichungstag und Bekanntmachung des Hinweises auf die Patenterteilung: **08.05.2013**
(21) Anmeldenummer: 10164760.0
(22) Anmeldetag: 02.06.2010
(51) Int. Cl.: F16K 7/06

(54) **Ventilvorrichtung zum Steuern eines Flusses eines Fluides durch einen Fluidkanal, Anordnung sowie Mehrwegventilvorrichtung**
Valve device for controlling a flow of a fluid through a fluid channel, assembly and multiple-way valve device
Dispositif de soupape pour la commande de l'écoulement d'un fluide à travers un canal de fluide, agencement et dispositif de soupape à voies multiples

(43) Veröffentlichungstag der Anmeldung: 07.12.2011
(73) Patentinhaber: Technische Universität Berlin, 10623 Berlin (DE); MT.DERM GmbH, 14167 Berlin (DE)
(72) Erfinder: Loth, Andreas, Dipl.-Ing., 13156 Berlin (DE); Bühs, Florian, Dipl.-Ing., 10777 Berlin (DE); Plückhahn, Kristian, Dipl.-Ing., 10318 Berlin (DE)
(74) Vertreter: Bittner, Thomas L.

(56) Entgegenhaltungen:
- EP-A1- 1 698 812
- WO-A1-93/16308
- WO-A1-96/08666
- DE-A1-102007 036 265
- US-A- 3 544 060
- US-A- 4 403 764
- US-A- 5 704 584

## Beschreibung

Die Erfindung betrifft eine Ventilvorrichtung zum Steuern eines Flusses eines Fluides durch einen Fluidkanal, eine Anordnung mit zumindest einer Ventilvorrichtung sowie eine Mehrwegventilvorrichtung.

### Hintergrund der Erfindung

Derartige Ventilvorrichtungen werden genutzt, um den Volumenstrom eines Fluides durch einen Fluidkanal einzustellen und zu verändern. Es ist in diesem Zusammenhang bekannt, den Fluss des Fluides durch den Kanal dadurch zu steuern, dass von außen auf einen Schlauch, in dem der Fluidkanal gebildet ist, Druck ausgeübt wird, wobei der Druck in Abhängigkeit von einem gewünschten Volumenstrom des Fluides durch den Kanal angepasst wird.

In der Medizintechnik erfolgt die Dosierung von Volumenströmen zum Beispiel über Mikroventile oder direkt über eine entsprechende Pumpe, mit der das abzugebende Fluid mit Druck beaufschlagt wird. Anwendung finden Ventile in der Medizin und im kosmetischen Bereich beispielsweise bei der Injektion von medizinisch und kosmetisch wirksamen Substanzen. Neben der Injektion von Substanzen zur Fettreduktion oder Faltenunterspritzung ist auch beim Farbeintrag für Tätowierungen oder permanentes Make-up im kosmetischen Bereich die dosierte Abgabe einer Substanz notwendig. Medizinische Anwendungen sind neben unterschiedlichen Impfungen auch zum Beispiel die Mesotherapie. Bei den genannten Anwendungen kann neben dem einfachen Eintrag von Medien auch die gleichzeitige Gabe von mehreren Medien vorgesehen sein, so dass diese erst nach dem Eintrag miteinander reagieren.

Das Dosieren des Fluides kann mittels einer berührenden oder einer berührungslosen Verfahren ausgefiihrt werden, die Fluidabgabeeinrichtung kommt also in Kontakt mit der Haut oder nicht. Berührungslose Geräte werden auch als so genannte Dispenser bezeichnet. Der Vorgang der Fluidabgabe selbst wird Dispensen, Jetten oder Pulsen genannt. Dieses Dosieren dient dem Eintrag von Substanzen auf die Haut oder in nachgeschaltete Substanzabgabesysteme (vgl. zum Beispiel EP 1 882 491). Berührend kann als das Dosieren auf eine Oberfläche oder durch diese hindurch verstanden werden.

Allen Anwendungen gemein ist der Bedarf einer präzisen Dosierung. Probleme bereiten oftmals chemische oder fluidische Eigenschaften, Partikel oder wechselnde Medien.

Das Dokument DE 103 37 484 B4 beschreibt ein berührungsloses Dosiersystem, bei dem ein Schlauch mit hoher Geschwindigkeit gequetscht wird, so dass sich ein frei fliegender Flüssigkeitstropfen bildet. Dosierfrequenzen von 50 Hz können auf diese Weise erreicht werden. Es handelt sich bei dem Aufbau um ein offenes System ohne Vordruck. Die Flüssigkeit befüllt den Schlauch aufgrund der Kapillarkräfte. Durch diesen Aufbau ist jedoch die maximale Dosiermenge und Dosierfrequenz beschränkt. Eine Funktion bei Gegendruck ist nicht oder nur sehr begrenzt möglich.

Im Dokument DE 693 25 591 T2 werden Ventilanordnungen zum Schalten einer Strömung durch flexible Schläuche beschrieben. Über einen schwenkbaren Hebel können zwei Positionen (bistabil auf / zu) angewählt werden. An die angekoppelten Flansche dieser mittels Gießen und Schweißen gefertigten Konstruktion sollen die Flüssigkeiten das Ventil durchströmen. Eine mögliche Kontamination der Flüssigkeit wird nicht verhindert, ebenso wenig wie sich das Prinzip nicht als Einwegteil oder für höhere Frequenzen (> 1 Hz) nutzen lässt.

Das Dokument EP 1 699 560 B1 beschreibt eine Möglichkeit, kleinste Mengen zu Pipetieren, basiert jedoch im Wesentlichen auf einer Kombination herkömmlicher Pipetiersysteme und dem bekannten Pipejet-Verfahren, also einer Schlauchdeformation, in diesem Fall als Pipettenspitze ausgeführt. Es ist so lediglich ein Dosieren kleinster Tropfen möglich, die frei fliegend zu ihrem Ziel gelangen. Für Injektionen lässt sich das Verfahren nicht nutzen, da ein Arbeiten bei Gegendruck nicht möglich ist.

Das Dokument DE 197 06 513 C2 beschreibt ein Mikrodosierverfahren, basierend auf einer Druckkammer mit Reservoiranschluss und Fluidauslass. Die Druckkammer wird durch einen Verdränger verkleinert, so dass Fluid zum Auslass gedrückt wird. Wesentlich ist hier eine Einrichtung zum Erfassen der Verdrängerposition.

Das Dokument US 2010/0030152 A1 beschreibt ein Mikronadel-Therapiesystem, bei dem anstelle einer Kanüle mehrere Kanülen verwendet werden.

Das Dokument EP 1 698 812 A1 beschreibt ein Ventil mit einem Ventilkörper und einem Schlauch aus einem Elastomer, wobei der Ventilkörper konfiguriert ist, mit Hilfe einer Rolle den Schlauch zu schließen bzw. zu öffnen.

Das Dokument WO 96/08666 A1 betrifft eine Durchflusskontrollvorrichtung mit einem verschiebbaren Element.

In dem Dokument WO 93/16308 A1 ist ein Ventil für einen Schlauch beschrieben, wobei der Fluss durch den Schlauch mit einem verlagerbaren Quetschelement eingestellt werden kann.

Das Dokument US 4 403 764 A beschreibt ein Verfahren und eine Vorrichtung zum Einstellen eines Flusses durch einen quetschbaren Schlauch.

Das Dokument DE 10 2007 036265 A1 betrifft eine Füllvorrichtung für ein fließfähiges Medium.

In dem Dokument US 3 544 060 A ist ein Ventil für einen Schlauch beschrieben, wobei der Durchmesser des Schlauches mit Hilfe eines Schraubelementes variiert werden kann.

Das Dokument US 5 704 584 A beschreibt ein Ventil für eine Infusionsvorrichtung.

### Zusammenfassung der Erfindung

Aufgabe der Erfindung ist es, verbesserte Technologien in Verbindung mit Ventilvorrichtungen zum Steuern eines Flusses eines Fluides durch einen Fluidkanal anzugeben, mit denen eine zuverlässige Steuerung des Volumenstroms des Fluides gewährleistet ist, insbesondere auch beim hochfrequenten Betätigen der Ventilvorrichtung.

Diese Aufgabe wird erfindungsgemäß durch eine Ventilvorrichtung zum Steuern eines Flusses eines Fluides durch einen Fluidkanal nach dem unabhängigen Anspruch 1 gelöst. Weiterhin sind vorgesehen eine Anordnung zumindest einer Ventilvorrichtung nach dem unabhängigen Anspruch 13 sowie eine Mehrwegeventilvorrichtung nach dem unabhängigen Anspruch 14. Vorteilhafte Ausgestaltungen der Erfindung sind Gegenstand von abhängigen Unteransprüchen.

Mit der erfindungsgemäßen Ventilvorrichtung ist die Möglichkeit geschaffen, den Volumenstrom durch den im Schlauch gebildeten Fluidkanal jeweiligen Anwendungserfordernissen entsprechend individuell einzustellen und zu regeln, insbesondere auch eine Volumenstromeinstellung mit hohen Veränderungsfrequenzen. Die aufgrund der Relativbewegung von Führung und Ventilbauteil bewirkte Verlagerung des Quetschelementes und die hierdurch erfolgte Veränderung des Durchflusses durch den Schlauch lässt sich in einer möglichen Anwendung auch hochfrequent als repetierende Bewegung ausführen. Die dem Ventilbauteil zugeordnete Führung kann dem jeweiligen Anwendungsfall genügend ausgestaltet werden, so dass ein ausreichender Verlagerungsweg für das Quetschelement bereitgestellt ist. So kann beispielsweise das Quetschelement aus einer ersten Stellung, in welcher der Fluidkanal in dem Schlauch vollständig geöffnet ist, in eine zweite Stellung verlagert werden, in welcher der Fluidkanal im Wesentlichen geschlossen ist. Bei dieser Ausgestaltung wird die Ventilvorrichtung in einer Nutzungsart als so genannter Schließer eingesetzt. Auch eine umgekehrte Verlagerung des Quetschelementes kann vorgesehen sein, was dann einer Ausführung als so genannter Öffner entspricht.

Eine Befestigung des Quetschelementes an dem Ventilbauteil lässt eine hochfrequente Verlagerung des Quetschelementes bei der Relativbewegung zum Öffnen und zum wenigstens teilweisen Schließen des Fluidkanals im Schlauch zu. Beispielsweise kann ein Kragarm vorgesehen sein.

Bevorzugt erfolgt die Verlagerung des Quetschelementes bei der Relativbewegung von Führung und Ventilbauteil im Wesentlichen quer zur Längsrichtung des Schlauches. Aber auch eine Bewegung des Quetschelementes auf einem Kreisbogenabschnitt kann beispielsweise vorgesehen sein.

Die Ventilvorrichtung kann in beliebigen Geräten oder Geräteteilen verwendet werden, um den Strom eines Fluides durch den Fluidkanal in einem Schlauch zu steuern. Insbesondere ist eine Verwendung in einer Dosiereinrichtung vorteilhaft, mit der auf dem Gebiet der Medizin oder der Kosmetik ein medizinischer oder kosmetischer Wirkstoff dosiert abzugeben ist. Die Dosiereinrichtung kann in berührender oder nicht berührender Bauart ausgeführt sein. Die Dosiereinrichtung kann zum Beispiel in eine Injektionsvorrichtung integriert sein, um die dosierte Abgabe eines Wirkstoffes bei Gegendruck zu steuern. Bei dieser Ausgestaltung ist die Ventilvorrichtung vorzugsweise einer den Wirkstoff abgebenden Kanüle nachgelagert. Die Kanüle sticht in die Haut ein und kann zum Beispiel bei einer vorgegebenen Stichtiefe den Wirkstoff abgeben. Die Dosiereinrichtung ist also nutzbar bei einem Verfahren zum dosierten Abgeben eines Fluides, insbesondere zum Injizieren in den menschlichen oder tierischen Körper.

Die Relativbewegung zwischen Führung und Ventilbauteil beim Einsatz der Ventilvorrichtung in einer beliebigen Dosiereinrichtung, die beispielsweise als Injektionseinrichtung ausgeführt ist, bei die Fluidabgabe auch gegen einen Gegendruck erfolgen kann, wird bevorzugt mittels Handbetätigung oder mit Hilfe eines Antriebes bewirkt. Beispielsweise verfügen Vorrichtungen zum Aufstechen einer menschlichen oder einer tierischen Haut über Antriebseinrichtungen, die mit hoher Frequenz eine Stecheinrichtung repetierend vor und zurück bewegen. Diese repetierende Bewegung kann dann für die Relativbewegung von Führung und Ventilbauteil genutzt werden, um den Fluidkanal im Schlauch zu Öffnen und zu Schließen. Solche Stechvorrichtungen sind beispielsweise beschrieben in DE 299 19 199 U1, EP 1 495 782 und EP 1 618 915. Weiterhin können beispielhaft die folgenden Dokumente genannt werden: EP 1 743 673, EP 2 149 388 und EP 1 882 492. Als Antriebsmechanismen können auch andere Technologien genutzt werden: Pneumatik, Hubmagnetantrieb, Antriebe mit rotierenden Motoren und Übersetzung in die Linearbewegung und Piezoantrieb. Die Erfindung ist in ihren verschiedenen Ausführungen insbesondere in Verbindung mit derartigen Stech- oder Injektionsvorrichtungen nutzbar.

Eine bevorzugte Weiterbildung der Erfindung sieht vor, dass das Ventilbauteil mit einem reversibel verlagerbaren Kragarm gebildet ist, an dem das Quetschelement angeordnet ist. Der Kragarm kann ein- oder mehrteilig ausgeführt sein. So können in einer Ausgestaltung mehrere Kragarme vorgesehen sein, die wahlweise zusammen wirken. Die Rückverlagerung erfolgt hierbei vorzugsweise aufgrund einer federelastischen Ausbildung des Kragarms. Beispielsweise erfolgt die reversible Verlagerung des Kragarms mittels einer Schwenkbewegung des Kragarmes. Das Quetschelement ist in einer Ausgestaltung an einem zur Basis des Kragarms, also einem Bereich, in welchem der Kragarm gelagert ist, distalen Ende gebildet. Bevorzugt ist das Quetschelement am Kragarm mit einem Vorsprung in Richtung des Schlauches gebildet, der von außen gegen den Schlauch drückt. Eine dem Schlauch zugeordnete Quetschoberfläche des Quetschelementes kann eine beliebige Oberflächenkontur aufweisen, zum Beispiel eine abgerundete Fläche, eine kugelförmige Oberfläche oder eine Oberfläche mit einer Quetschkante, die im Bereich von zwei schräg aufeinander zulaufenden Oberflächen gebildet ist. Auch eine Kombination solcher Oberflächenkonturen kann vorgesehen sein, insbesondere zur Optimierung der Quetschwirkung.

Bei einer zweckmäßigen Ausgestaltung der Erfindung kann vorgesehen sein, dass sich der Kragarm längs des Schlauches erstreckt und mit der Längsrichtung des Schlauches wahlweise einen spitzen Winkel einschließt. In einer Ausführungsform verläuft der Kragarm im Wesentlichen parallel zur Längsrichtung des Schlauches.

Eine vorteilhafte Ausführungsform der Erfindung sieht vor, dass der Führungsabschnitt an dem Quetschelement gebildet ist. Bei dieser Ausführungsform übernimmt das Quetschelement zusätzlich die Funktionalität des Zusammenwirkens von Ventilbauteil und der dem Ventilbauteil zugeordneten Führung. Das Quetschelement ist dann als integriertes Element, nämlich als kombiniertes Quetsch- und Führungselement ausgeführt, an dem der Führungsabschnitt gebildet ist. Beispielsweise gleitet in einer Ausführungsform bei der Relativbewegung von Ventilbauteil und Führung ein Oberflächenabschnitt des Quetschelementes entlang der dem Ventilbauteil zugeordneten Führung.

Bevorzugt sieht eine Fortbildung der Erfindung vor, dass die Führung an einen Wandabschnitt gebildet ist. Der Wandabschnitt kann beispielsweise an einem Gehäuse gebildet sein, in welchem Ventilbauteil und Schlauch aufgenommen sind. Eine Ausführungsform sieht dann vor, dass ein Gehäuseteil mit dem hieran gebildeten Wandabschnitt im Betrieb relativ zum Ventilbauteil bewegt wird und der Führungsabschnitt des Ventilbauteils hierbei entlang der Führung an dem Gehäuseteil gleitet, wodurch das Quetschelement verlagert wird, was zur Veränderung des Flusses durch den Fluidkanal führt. Umgekehrt kann vorgesehen sein, das Ventilbauteil in dem Gehäuse zu bewegen, so dass wiederum der Führungsabschnitt entlang der an dem Gehäuseteil gebildeten Führung gleitet, was zur Verlagerung des Quetschelementes führt. Auch können sich sowohl Ventilbauteil als auch Wandabschnitt mit hieran gebildegebildeter Führung bewegen. In dieser oder anderen Ausführungsformen ist vorgesehen, die Verlagerung des Quetschelementes und somit die Veränderung des Flusses durch den Fluidkanal repetierend mit einer Frequenz zwischen etwa 10 Hz und etwa 500 Hz, bevorzugt zwischen etwa 50 Hz und etwa 150 Hz, auszuführen. Es ist alternativ auch die Verwendung in einem Einzelstichmodus denkbar, bei dem jeweils nur ein einzelner Einstich oder eine Injektion nach manuellen Auslösen durch den Anwender erfolgt.

Bei einer vorteilhaften Ausgestaltung der Erfindung kann vorgesehen sein, dass das Ventilbauteil als ein Mikroventilbauteil gebildet ist. Auf diese Weise ist die Ventilvorrichtung beispielsweise für eine Mikrodosiereinrichtung.

Eine Weiterbildung der Erfindung kann vorsehen, dass das Quetschelement den Schlauch wenigstens hälftig umgreifend gebildet ist.

Eine bevorzugte Weiterbildung der Erfindung sieht vor, dass das Quetschelement mit mehreren Teilquetschelementen gebildet ist, die um den herum Schlauch angeordnet sind. In einer Ausführung sind die mehreren Teilquetschelemente den Schlauch im Wesentlichen vollständig umgreifend gebildet. Eine Ausführungsform sieht vor, dass die Quetschelemente an einer gemeinsamen Basis angeordnet sind. Es kann vorgesehen sein, dass die mehreren Teilquetschelemente aufgrund der Relativbewegung von Führung und Ventilbauteil gemeinsam bewegt werden. Diese Bewegung kann gleichartig und / oder gleichzeitig erfolgen. In einer bevorzugten Ausgestaltung sind die mehreren Teilquetschelemente rotationssymmetrisch um den Schlauch herum gebildet. In einer Fortbildung der Erfindung ist die Anordnung der mehreren Teilquetschelemente im Wesentlichen einem Druckminen-Mechanismus entsprechend ausgeführt.

Bei einer zweckmäßigen Ausgestaltung der Erfindung kann vorgesehen sein, dass die zumindest ein Teil der mehreren Teilquetschelemente wenigstens in einer Endstellung aufeinander lagernd angeordnet sind. Hierbei kann vorgesehen sein, dass in der Endstellung, in welcher mittels Quetschen des Schlauches der Fluidkanal teilweise oder vollständig geschlossen ist, einzelne Teilquetschelemente sich aufeinander abstützen, so dass in einer Ausführungsform ein weiteres Schließen des Fluidkanals nicht mehr möglich ist.

Bevorzugt sieht eine Fortbildung der Erfindung vor, dass das Ventilbauteil wenigstens zum Teil als ein Spritzgussteil ausgeführt ist. Spritzgussteile sind kostengünstig und auch in Massenfertigung herstellbar. In dieser oder anderen Ausführungsformen kann vorgesehen sein, das Ventilbauteil als Einwegartikel auszuführen. Die Spritzgussausfülhrung des Ventilbauteils kann in einer Weiterbildung in Zwei-Komponenten-Spritzgießen (2K-Spritzgießen) erfolgen. Die Ventilvorrichtung mit dem wenigstens dem Ventilbauteil, einem Schlauchabschnitt, dem Führungsabschnitt und wahlweise einer an den Schlauchabschnitt gekoppelten Kanüle kann in einer Ausgestaltung als Einwegmodul oder Einmalmodul ausgeführt sein. Dieses Einmalmodul kann in einem einfachen Schritt an eine Antriebseinrichtung gekoppelt werden.

Bei einer vorteilhaften Ausgestaltung der Erfindung kann vorgesehen sein, dass das Ventilbauteil auf dem Schlauch angeordnet ist. In einer Ausführungsform ist das Ventilbauteil auf dem Schlauch sitzend gebildet, bevorzugt lösbar. Zum Beispiel erfolgt die Anordnung des Ventilbauteils auf den Schlauch mittels einer elastischen Klemmwirkung, die bevorzugt von dem Quetschelement und einem dem Quetschelement zugeordneten Gegenstück bereitgestellt ist. Eine Weiterbildung sieht vor, dass das Ventilbauteil in dieser Ausgestaltung mit einer Schlauchführung gebildet ist, entlang welcher der Schlauch wenigstens abschnittsweise verläuft, wenn das Ventilbauteil auf diesem angeordnet wird. In einer Ausgestaltung verlaufen die Schlauchführung und der Kragarm mit dem Quetschelement im Wesentlichen parallel in Längsrichtung des Schlauches, zum Beispiel oberhalb und unterhalb des Schlauches.

Eine Weiterbildung der Erfindung kann vorsehen, dass der Fluidkanal durch einen die Relativbewegung von Führung und Ventilbauteil wenigstens teilweise ausgleichenden Ausgleichsabschnitt verläuft. Mit Hilfe des Ausgleichsabschnittes wird eine Verlagerung des Ventilbauteils mit einem hieran fest angeordneten Schlauchabschnitt bei der Relativbewegung in Bezug auf einen feststehendes Schlauchabschnitt toleriert, beispielsweise mittels eines Schlauchabschnitts mit einer oder mehreren offenen Schlaufen. Ist der Ausgleichsabschnitt zum Beispiel einer Kanüle nachgelagert, so kann auf diese Weise eine repetierende Stechbewegung der Kanüle ausgeglichen werden. Eine solche Ausführung kann insbesondere in einer Dosier- oder einer Injektionseinrichtung zum dosierten Abgeben eines Fluides, beispielsweise eines medizinischen oder eines kosmetischen Wirkstoffes, vorgesehen sein.

In Verbindung mit der Anordnung mit zumindest einer Ventilvorrichtung sowie einer mit dem Fluidkanal in Fließverbindung stehender Druckbeaufschlagungseinrichtung kann die Integration dieser Anordnung in eine Dosiereinrichtung zum dosierten Abgeben eines Fluides vorgesehen sein, insbesondere eines medizinischen oder kosmetischen Stoffes. Die Fluidabgabe kann hierbei unter Einfluss eines Gegendrucks erfolgen, was insbesondere in Verbindung mit Injektionen von Bedeutung ist. Beispielsweise wird eine solche Injektionseinrichtung in Verbindung mit einer Vorrichtung zum Tätowieren oder zum Ausbilden von permanentem Make-up verwendet. Hierbei wird mit der Ventileinrichtung die Abgabe eines Farbstoffes gesteuert. Aber auch die Dosierung eines anderen kosmetischen Wirkstoffes oder eines medizinischen Stoffes kann vorgesehen sein.

Bei der Mehrwegeventilvorrichtung kann in einer Ausgestaltung vorgesehen sein, dass nebeneinander benachbart angeordnete Fluidkanäle hinsichtlich des sie jeweils durchströmenden Flusses eingestellt werden. Bei einer Ausgestaltung ist ein integriertes Ventilbauteil geschaffen, welches zur Steuerung des Volumenstromes in mehreren Fluidkanälen dient. Zu diesem Zweck verfügt das integrierte Ventilbauteil über mehrere Quetschelemente, die jeweils einem Schlauch mit hierin gebildetem Fluidkanal zugeordnet sind. In der Mehrwegeventilvorrichtung können auch Kreuzungen zwischen Fluidkanälen ausgebildet sein.

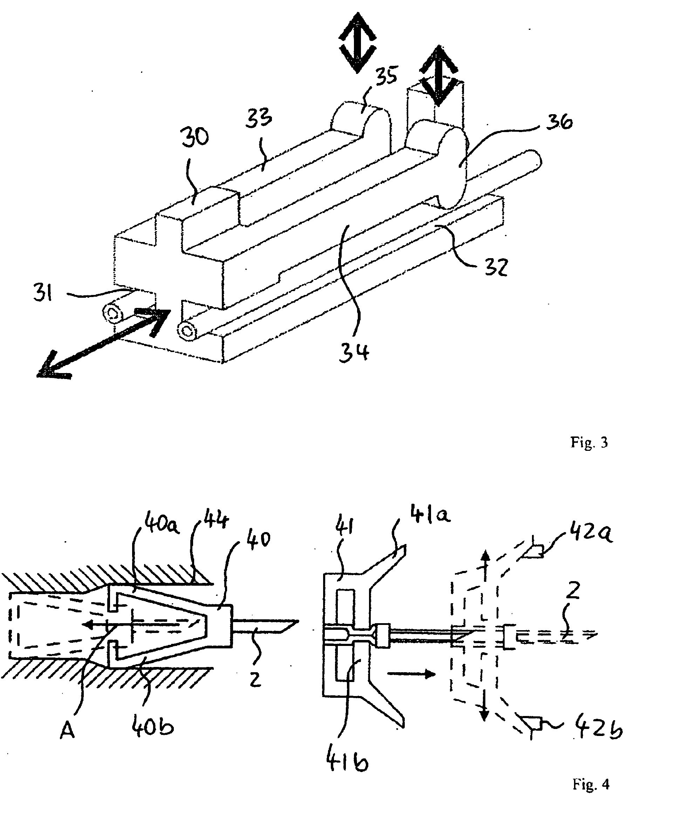

Eine Hintereinanderschaltung mehrerer Ventilbauteile kann geeignet sein, die Dosiergenauigkeit zu erhöhen, insofern als das beispielsweise das Volumen zwischen den Ventilbauteilen ein Fluid unter Druck enthält und durch geschicktes Öffnen und Schließen nur das zwischengespeicherte Volumen abgegeben wird. Beispielsweise kann ein entsprechendes Ventil aus Schläuchen unterschiedlicher Wandstärke oder aus Schlauch Rohrkombinationen bestehen. Der "speichernde" Schlauchabschnitt muss lediglich in der Lage sein, Energie zu speichern die einen Überdruck in diesem Abschnitt aufrechterhält. (Ballon mit einem Ein- und Auslass). Dies ist insbesondere für die rein mechanische Ausführung von Interesse. Die Verschaltung mehrerer Ventilbauteile ist möglich und kann beispielsweise genutzt werden, um Mehrwegeventile darzustellen (vgl. Fig. 3). Nach dem Ventilbauteil können sich also auch weitere Bauteile anschließen.

Die Ventilvorrichtung kann weiterhin zum An- oder Absaugen verwendet werden. Auch einen Kombination von beidem kann vorgesehen sein, beispielsweise ein Ansaugen und ein Ausstoßen im Wechsel oder mehrstufig hintereinander (beispielsweise: An, An, Aus, Aus, Aus, Aus usw.). Dies kann zum Beispiel für die Tattooentfernung genutzt werden, bei der Wirksubstanzen in das Tattoo injiziert werden und eine Absaugung der Tattoopigmente erfolgt. Diese Methode der Tattooentfernung ist als solche zum Beispiel im Dokument WO 2005/020828.

Ein Vorteil der Erfindung ist die Möglichkeit, vordosierte oder wechselnde und oder auskristallisierende / eintrocknende Medien zu dosieren, ohne die Anlage zu beschädigen oder reinigen zu müssen, da der Fluidtransport nur in einem leicht zu wechselnden System stattfindet.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Darstellung einer Anordnung mit einer Ventilvorrichtung,
- Fig. 2: mehrere Ausgestaltungen für ein Ventilbauteil,
- Fig. 3: eine schematische Darstellung einer Mehrwegeventilvorrichtung, bei der zwei Fluidkanäle parallel zueinander verlaufen,
- Fig. 4: eine schematische Darstellung einer Anordnung mit einer weiteren Ventilvorrichtung und
- Fig. 5: eine schematische Darstellung eines Ventilbauteils mit mehreren Teilquetschelementen.

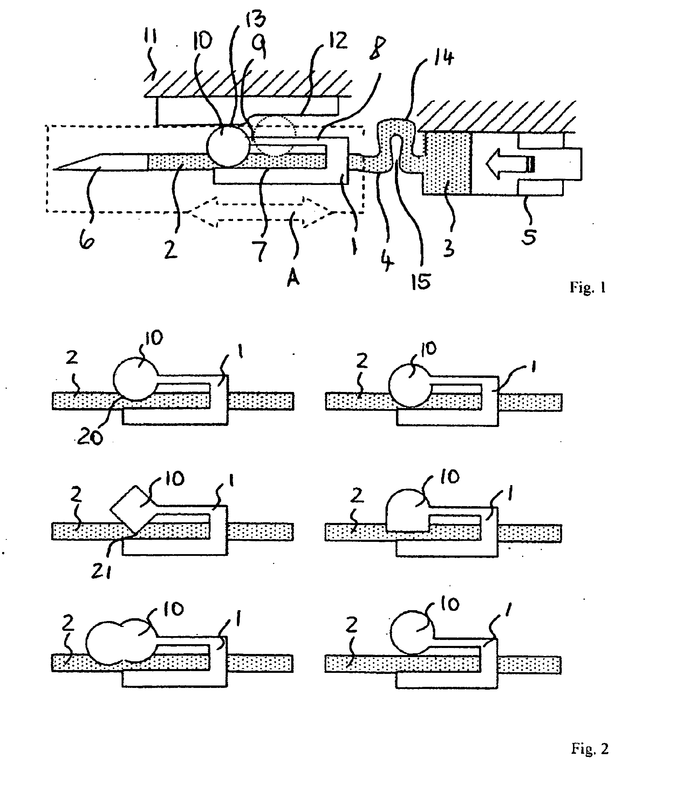

Fig. 1 eine schematische Darstellung einer Anordnung mit einem Ventilbauteil 1, welches auf einem Schlauch 2 angeordnet ist, in dem für ein Fluid 3 ein Fluidkanal 4 gebildet ist, durch welchen das Fluid 3 aus einem mit Druck beaufschlagten Reservoir 5 zu einer Kanüle 6 gelangt, wo das Fluid 3 in dosierter Form abgeben wird. Das Reservoir 5 kann beispielsweise ein Reservoir für Farben sein, die zum Ausbilden eines Tattoos oder von permanentem Make-up dienen. Auch kann das Reservoir in einer anderen Ausgestaltung zum Beispiel eine Substanz zur Faltenunterspritzung enthalten.

Das Ventilbauteil 1 ist mit einer Schlauchführung 7 gebildet, auf welcher der Schlauch 2 aufliegt. Der Schlauchführung 7 gegenüberliegend ist ein Kragarm 8 gebildet. Am distalen Ende 9 des Kragarms 8 ist ein Quetschelement 10 angeordnet, welches von außen auf den Schlauch 2 drückt, wenn das Ventilbauteil 1 in der in Fig. 1 dargestellten Relativposition bezüglich eines Wandabschnittes 11 mit einer hieran gebildeten Führung 12 angeordnet ist. Wird das Ventilbauteil 1 (vgl. Pfeil A in Fig. 1) relativ zur Führung 12 in Längsrichtung des Schlauches 2 bewegt, so gleitet ein Führungsabschnitt 13 an dem Quetschelement 10 entlang der Führung 12. Auf diese Weise wird das Quetschelement 10 quer zur Längsrichtung des Schlauches 2 verlagert, wodurch der in dem Schlauch 2 gebildete Fluidkanal 4 geöffnet und geschlossen wird. Die Relativbewegung zwischen Ventilbauteil 1 und Führung 12 ermöglicht so eine dosierte Abgabe des Fluides 3 durch die Kanüle 6.

In einem dem Ventilbauteil 1 nachgelagerten Bereich ist ein Ausgleichsabschnitt 14 gebildet, welcher die Bewegung des Ventilbauteils 1 und des hierdurch mitbewegten Schlauchabschnitts relativ zum Reservoir 5 ausgleicht. Zu diesem Zweck bildet der Schlauch 2 im Ausgleichsabschnitt 14 eine offene Schlaufe 15.

Mittels des jeweiligen Quetschelementes lässt sich für ein bekanntes Medium der Volumenstrom einstellen, indem der anliegende Druck, die Öffnungsweite und / oder ―zeit geregelt werden. Diese Parameter beeinflussen den Umfang an dosierbaren Substanzen beziehungsweise Viskositäten. Prinzipiell lassen sich alle flüssigen und gasförmigen Substanzen mit im Vergleich zum Schlauchinnendurchmesser nicht zu großen Partikeln dosieren.

Die Betätigung der jeweiligen Klemm- oder Quetschvorrichtung lässt sich rein mechanisch, durch eine Bewegung der Klemmvorrichtung in eine Führung mit entsprechender Wandgestaltung, die ein Öffnen oder Schließen der mittels des Quetschelementes gebildeten Klemme hervorruft, erzeugen. Die Rückstellung kann über die elastische Verformung und oder Rückformkräfte des Schlauchs und / oder durch den Druck des im Schlauch befindlichen Mediums auf die Schlauchwand erzielt werden. Auf diese Weise kann ein Dosieren bei hohen Frequenzen erfolgen.

Das Ventilbauteil mit dem Quetschelement kann fest mit dem Schlauch verbunden sein oder an diesem nachträglich befestigt werden. In letzterem Fall kann die Ventilvorrichtung als Mehrwegventil ausgeführt sein. Aufgrund sehr geringer Herstellungskosten, beispielsweise im Spritzgießverfahren aus Kunststoff und der besseren Handhabbarkeit, ist eine Einwegnutzung vorzuziehen, aber auch Varianten aus anderen Materialien wie Metall, Verbundwerkstoffe oder dergleichen sind möglich.

Das Ventilbauteil kann in seinen verschiedenen Ausführungen als Öffner oder Schließer arbeiten. Eine stufenlose Veränderung des Volumenstroms kann gebildet sein. Die Druckbeauschlagung des Schlauches kann ein- oder mehrseitig erfolgen. Unter einseitig wird hier verstanden, dass der Volumenquerschnitt von einer Seite aus begrenzt wird, beispielsweise drückt das Quetschelement von einer Seite auf einen gegenüber dem Quetschelement auf einen Untergrund fixierten Schlauch. Im zweiseitigen Fall ist das Ventilbauteil nach dem Prinzip einer Zange denkbar (vgl. Fig. 4), bei der Klemmbacken gegeneinander geführt werden.

Fig. 2 zeigt mehrere Ausführungsformen für das Ventilbauteil 1, wobei das Quetschelement 10 unterschiedlichen geometrischen Gestaltungen entsprechend gebildet ist. Insbesondere eine Flächenform in einem dem Schlauch 2 zugewandten Quetschabschnitt 20 des Quetschelementes 10 ist auf unterschiedliche Art und Weise gestaltet. Hierzu gehören eine abgerundete Fläche, eine kugelförmige Fläche, eine ebene Fläche sowie ein Flächenbereich mit einer Quetschkante 21.

Fig. 3 zeigt eine schematische Darstellung einer Mehrwegeventilvorrichtung, bei der ein Doppelventilbauteil 30 mit parallelen Schlauchführungen 31, 32 gebildet ist. An jeweiligen Kragarmen 33, 34 ist ein zugehöriges Quetschelement 35, 36 gebildet, welches auf den zugeordneten Schlauch von außen drückt.

Fig. 4 zeigt eine schematische Darstellung von zwei Ventilbauteilen (vgl. rechte und linke Seite in Fig. 4), bei denen das jeweilige Quetschelement 40, 41 mit zugeordneten Klemmbacken 40a, 40b und 41a, 41b gebildet ist, die bei einer Relativbewegung des jeweiligen Ventilbauteils 40, 41 entlang einer Führung 44 aufeinander zu beziehungsweise voneinander weg bewegt werden. Bei der Ausführungsform auf der linken Seite in Fig. 4 sind die Klemmbacken 40a, 40b in einer Ausgangsstellung voneinander beabstandet und gegen ein Zusammendrücken vorgespannt. Bewegt sich das Ventilbauteil 40 entlang des Pfeils A so werden die Klemmbacken 40a, 40b aufeinander zu bewegt, wodurch Druck auf den zwischen den Klemmbacken 40a, 40b angeordneten Schlauch 2 ausgeübt wird. Die gestrichelten Linien auf der linken Seite in Fig. 4 zeigen einen Zustand, in welchem die Klemmbacken 40a, 40b den Schlauch 2 mit dem Fluidkanal schließen.

Bei der Darstellung auf der rechten Seite in Fig. 4 zeigen die gestrichelten Linien eine Stellung des Ventilbauteils 41, in welcher die Klemmbacken 41a, 41b mit Hilfe von Anschlägen 42a, 42b auseinander gedrückt werden, so dass der Fluidkanal im Schlauch 2 freigegeben ist.

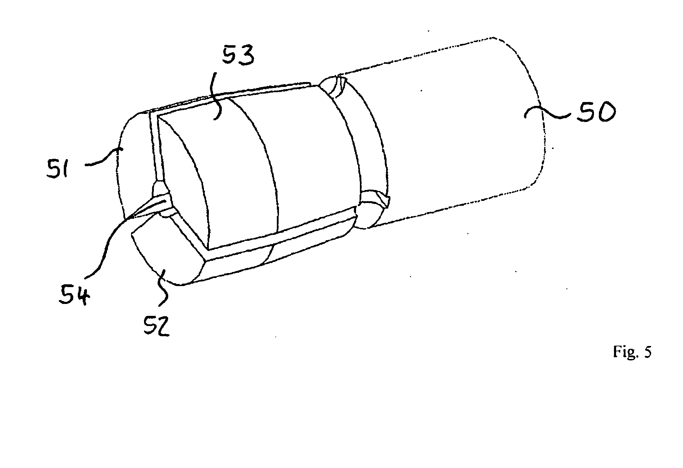

Fig. 5 zeigt eine schematische Darstellung eines Ventilbauteils 50, bei dem mehrere Teilquetschelemente 51, 52, 53 um eine Schlauchführung 54 herum angeordnet. Das Ventilbauteil 50 ist für eine Art "Druckminen-Mechanismus" gebildet, bei dem aufgrund der Relativbewegung von Ventilbauteil 50 und Führung (nicht dargestellt in Fig. 5) die Teilquetschelemente 51, 52, 53 aufeinander zu (Schließen des Schlauches) oder voneinander weg (Öffnen des Schlauches) bewegt werden.

In ersten Untersuchungen konnten bereits Abgabefrequenzen von bis zu 100 Hz erreicht werden. Die dosierten Tröpfchen lösten sich dabei sowohl in waagerechter als auch in senkrechter Lage vollständig vom Schlauchende. Als Testflüssigkeit wurde destilliertes Wasser verwendet, bei einem Druck von 2 bar und einem Schlauchdurchmesser von 0,7 X 0,3 mm (außen zu innen). Das saubere Ablösen der einzelnen Tröpfchen wurde mit einer High-Speed-Kamera bei einer Aufzeichnungsfrequenz von 1.500 Hz überprüft. Der Versuchsaufbau wurde bereits im miniaturisierten Maßstab aus Kunststoff hergestellt. Die Abmaße des funktionsfähigen Ventils liegen bei 4x4x15 mm.

Anwendungsbereiche finden sich vor allem in der Injektion von medizinischen und kosmetischen Substanzen. Die Integration in ein Bündel Vollnadeln einer Stech- oder Injektionsvorrichtung kann ebenso vorgesehen sein wie mehrere Kanülen gleichzeitig zu betreiben. Als Beispiele für die kosmetische Behandlungen lassen sich die Carboxytherapie also die Injektion von C02, zum Beispiel zur Fettreduktion, Faltenunterspritzung in Tiefen von 1,0 bis 4,0 mm mit verschiedensten Medien, Tattooeintrag und deren Entfernung in Tiefen von 1,0 - 3,5 mm oder Permanent Make-up Eintragung in Tiefen von 0,3 - 1,0 mm nennen. Sowohl zur kosmetischen als auch zur medizinischen Anwendung ist die Absaugung aus der Haut im Allgemeinen denkbar. Rein medizinische Anwendungen sind neben unterschiedlichten Impfungen in Tiefen von 0,2 - 0,6 mm die Mesotherapie in Tiefen von 0,2 - 10 mm.

Die nachfolgende Liste stellt eine Auswahl möglicher Substanzen dar, die eingebracht werden können: Hyaluron-Säure, Vitamin, Q10, Vakzin, therapeutische Antikörper, Krebs-Antikörper, Diabetes-Therapeutikum, Hormon, Zytokine, biochemischer oder biologischer Signalstoff, Anti-Oxidant, Haarwuchsmittel, Haarwachstumshemmer, Mineralstoffe zur Verbesserung der Hautspannung und eines Hautmetabolismus, Enzyme, Co-Enzym, Aminosäure, Nukleinsäure, inerte Farbpartikel, inerter Hautfüller, Nerven aktivierender Wirkstoff wie Botox oder bakterielles Toxin, diabetisches Kontrollmittel wie von der Glycosekonzentration abhängig farbveränderliche Partikel.

Für alle Anwendung ist neben dem einfachen Eintrag von Medien auch die gleichzeitige Gabe von mehreren Medien denkbar, die beispielsweise erst nach der Injektion miteinander reagieren sollen. Ein Teil der Anwendungen hat ein Einbringen oder Entfernen unterschiedlichster Medien in definierter Tiefe beziehungsweise zu einem bestimmten Zeitpunkt gemein. Gerade in der intrakutanen Impftechnik wird deutlich, welchen Stellenwert die genaue Tiefe der Impfung besitzt. So verliert der zu impfende Wirkstoff bereits beim Überschreiten der Zieltiefe um mehr als 15% einen erheblichen Anteil seiner Wirkung.

Neben dem Verpacken kleinster Mengen eines Mediums, wie teuren Medikamenten, lässt sich das gewünschte Mischverhältnis, beispielsweise mit mehreren unterschiedlichen Schlauchdurchmessern, auf einfachste Weise einstellen.

Vor allem in Bereichen wie der Impftechnik sind selbst schwer zu handhabende Reagenzien einfach einsetzbar, da diese in einem geschlossenen Behältnis mit Ventil verwendet werden können und so mit dem eigentlichen Dispenser nicht in Kontakt kommen. Auf ein umständliches Reinigen der Geräte kann daher verzichtet werden.

Es ist weiterhin denkbar, eine Verschlussflüssigkeit, vergleichbar mit einem Korken, beispielsweise eine hydrophile Wirksubstanz und hydrophobe Verschlusssubstanz m verwenden und so ein Auslaufen der eingebrachten Substanz zu verhindern. Auch eine hochviskose Verschlusssubstanz kann zu diesem Zweck verwendet werden. Alternativ lässt sich der Verschluss der Haut durch Koagulieren dieser mit HF, Laser oder anderen Thermoeinwirkungen realisieren.

Die Erfindung in ihren verschiedenen Ausgestaltungen lässt sich mit weiteren Technologien kombinieren, beispielsweise mit der Messung der Tiefe der Hautschichten und der automatischen Einstellung der optimalen Stichtiefe der Nadel. Die Substanzen können exakt in die vordefinierten Hautschichten injiziert werden. Die Mess- und Stichtiefentechnologie ist beispielsweise im Dokument EP 1 882 492 beschrieben.

Aufgrund der einfachen Konstruktion, der vielfältigen Betätigungsvarianten und der geringen Baugröße, lässt sich die Erfindung mit minimalem Aufwand in eine Vielzahl unterschiedlicher Produkte integrieren.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein, jedoch im rahmen der Ansprüche.

## Patentansprüche

1. Ventilvorrichtung zum Steuern eines Flusses eines Fluides durch einen Fluidkanal, mit:
- einem Schlauch aus flexiblem Material, in welchem ein Abschnitt eines Fluidkanals gebildet ist,
- einem dem Schlauch zugeordneten Ventilbauteil (1; 40; 41; 50),
- einem Quetschelement (10; 40a, 40b; 41a, 41b; 51, 52, 53), welches an dem Ventilbauteil (1; 40; 41; 50) gebildet und konfiguriert ist, mittels Drücken gegen eine Außenfläche des Schlauches (2) einen Fluss eines Fluides durch den Fluidkanal zu steuern,
- einem Führungsabschnitt, welcher an dem Ventilbauteil (1; 40; 41; 50) gebildet ist, und
- einer dem Ventilbauteil (1; 40; 41; 50) zugeordneten Führung, welche bei einer Relativbewegung von Führung und Ventilbauteil (1; 40; 41; 50) relativ zueinander in Längsrichtung des Schlauches (2) mit dem Führungsabschnitt zusammenwirkt, derart, dass bei der Relativbewegung das Quetschelement (10; 40a, 40b; 41a, 41b; 51, 52, 53) den Druck auf die Außenfläche des Schlauches (2) und somit den Fluss durch den Fluidkanal (4) verändernd verlagert wird,
**dadurch gekennzeichnet, dass** das Quetschelement (10; 40a, 40b; 41 a, 41b; 51, 52, 53) mittels einer Befestigung an dem Ventilbauteil (1; 40; 41; 50) angeordnet ist, die eine hochfrequente repetierende Verlagerung des Quetschelementes (10; 40a, 40b; 41a, 41b; 51, 52, 53) mit einer Frequenz zwischen 10Hz, und 500Hz bei der Relativbewegung zum Öffnen und zum wenigstens teilweisen Schließen des Fluidkanals im Schlauch (2) zulassend gebildet ist.

2. Ventilvorrichtung nach Anspruch 1, **dadurch gekennzeichnet, dass** das Ventilbauteil (1; 40; 41; 50) mit einem reversibel verlagerbaren Kragarm gebildet ist, an dem das Quetschelement (10; 40a, 40b; 41a, 41b; 51, 52, 53) angeordnet ist.

3. Ventilvorrichtung nach Anspruch 2, **dadurch gekennzeichnet, dass** sich der Kragarm längs des Schlauches (2) erstreckt und mit der Längsrichtung des Schlauches (2) wahlweise einen spitzen Winkel einschließt.

4. Ventilvorrichtung nach mindestens einem der vorangehenden Ansprüche, dadurch g e - k**ennzeichnet**, dass der Führungsabschnitt an dem Quetschelement (10; 40a, 40b; 41 a, 41b; 51, 52, 53) gebildet ist.

5. Ventilvorrichtung nach mindestens einem der vorangehenden Ansprüche, dadurch g e - **kennzeichnet**, dass die Führung an einen Wandabschnitt gebildet ist.

6. Ventilvorrichtung nach mindestens einem der vorangehenden Ansprüche, dadurch g e - **kennzeichnet,** dass das Ventilbauteil als ein Mikroventilbauteil gebildet ist.

7. Ventilvorrichtung nach mindestens einem der vorangehenden Ansprüche, dadurch g e - **kennzeichnet**, dass das Quetschelement (40a, 40b; 41a, 41b; 51, 52, 53) den Schlauch (2) wenigstens hälftig umgreifend gebildet ist.

8. Ventilvorrichtung nach mindestens einem der vorangehenden Ansprüche, dadurch g e - **kennzeichnet,** dass das Quetschelement mit mehreren Teilquetschelementen (40a, 40b; 41a, 41b; 51, 52, 53) gebildet ist, die um den Schlauch (2) herum angeordnet sind.

9. Ventilvorrichtung nach Anspruch 8, **dadurch gekennzeichnet, dass** zumindest ein Teil der mehreren Teilquetschelemente (51, 52, 53) wenigstens in einer Endstellung aufeinander lagernd angeordnet sind.

10. Ventilvorrichtung nach mindestens einem der vorangehenden Ansprüche, dadurch g e - **kennzeichnet,** dass das Ventilbauteil (1; 40; 41; 50) wenigstens zum Teil als ein Spritzgussteil ausgeführt ist.

11. Ventilvorrichtung nach mindestens einem der vorangehenden Ansprüche, dadurch g e - **kennzeichnet,** dass das Ventilbauteil (1; 40; 41; 50) auf dem Schlauch (2) angeordnet ist.

12. Ventilvorrichtung nach mindestens einem der vorangehenden Ansprüche, dadurch g e - **kennzeichnet**, dass der Fluidkanal durch einen die Relativbewegung von Führung und Ventilbauteil wenigstens teilweise ausgleichenden Ausgleichsabschnitt (14) verläuft.

13. Anordnung mit zumindest einer Ventilvorrichtung nach mindestens einem der vorangehenden Ansprüche und einer mit dem Fluidkanal in Fließverbindung stehender Druckbeaufschlagungseinrichtung, die konfiguriert ist, das Fluid in dem Fluidkanal mit einem Druck zu beaufschlagen.

14. Mehrwegeventilvorrichtung mit mehreren Ventilvorrichtungen nach mindestens einem der Ansprüche 1 bis 12.

## Claims

1. A valve for controlling a flow of a fluid through a fluid channel, comprising:
- a tube of flexible material in which a section of a fluid channel is formed,
- a valve component (1; 40; 41; 50) assigned to the tube,
- a squeeze element (10; 40a, 40b; 41a, 41b; 51, 52, 53), which is formed on the valve component (1; 40; 41; 50) and which is configured to control a flow of a fluid through the fluid channel by pressing against an outside surface of the tube (2),
- a guide section, which is formed on the valve component (1; 40; 41; 50), and
- a guide assigned to the valve component (1; 40; 41; 50), which cooperates with the guide section in a relative movement of the guide and valve components (1; 40; 41; 50) relative to one another in the longitudinal direction of the tube (2), such that the squeeze element (10; 40a, 40b; 41a, 41b; 51, 52, 53) is displaced in the relative movement, changing the pressure on the outside surface of the tube (2) and thus changing the flow through the fluid channel,
**characterized in that** a fastening of the squeeze element (10; 40a, 40b; 41a, 41b; 51, 52, 53) and the valve component (1; 40; 41; 50) is formed so that it allows a highfrequency displacement of the squeeze element (10; 40a, 40b; 41a, 41b; 51, 52, 53) in a relative movement to open and at least partially close the fluid channel in the tube (2) with a frequency between 10 Hz and 500 Hz.

2. The valve according to claim 1, **characterized in that** the valve component (1; 40; 41; 50) is formed with a reversibly displaceable cantilevered arm on which the squeeze element (10; 40a, 40b; 41a, 41b; 51, 52, 53) is arranged.

3. The valve according to claim 2, **characterized in that** the cantilevered arm extends along the tube (2) and optionally forms an acute angle with the longitudinal direction of the tube (2).

4. The valve according to at least one of the preceding claims, **characterized in that** the guide section is formed on the squeeze element (10; 40a, 40b; 41a, 41b; 51, 52, 53).

5. The valve according to at least one of the preceding claims, **characterized in that** the guide is formed on a wall section.

6. The valve according to at least one of the preceding claims, **characterized in that** the valve component is formed as a microvalve component.

7. The valve according to at least one of the preceding claims, **characterized in that** the squeeze element (40a, 40b; 41a, 41b; 51, 52, 53) is formed, extending around at least half of the tube (2).

8. The valve according to at least one of the preceding claims, **characterized in that** the squeeze element is formed with a plurality of partial squeeze elements (40a, 40b; 41 a, 41b; 51, 52, 53), which are arranged around the tube (2).

9. The valve according to Claim 8, **characterized in that** the at least a part of the plurality of partial squeeze elements (51, 52, 53) is arranged so they are supporting one another in at least one end position.

10. The valve according to at least one of the preceding claims, **characterized in that** the valve component (1; 40; 41; 50) is designed at least in part as an injection-molded part.

11. The valve according to at least one of the preceding claims, **characterized in that** the valve component (1; 40; 41; 50) is arranged on the tube (2).

12. The valve according to at least one of the preceding claims, **characterized in that** the fluid channel runs through a compensating section (14), which at least partially compensates for the relative movement of the guide and the valve component.

13. A system having at least one valve according to at least one of the preceding claims and a pressure-applying device which is in fluid connection with the fluid channel and is configured to apply a pressure to the fluid in the fluid channel.

14. A multiple-way valve having a plurality of valves according to at least one of the claims 1 to 12.

## Revendications

1. Dispositif de soupape pour la commande de l'écoulement d'un fluide à travers un canal à fluide, avec :
- un tuyau en matière souple, dans lequel est formée une section d'un canal à fluide,
- une pièce de soupape (1 ; 40 ; 41 ; 50) attribuée au tuyau,
- un élément de serrage (10 ; 40a, 40b ; 41a, 41b ; 51, 52, 53) formé sur la pièce de soupape (1 ; 40 ; 41 ; 50) et configuré pour commander l'écoulement d'un fluide à travers le canal à fluide au moyen d'une pression contre une surface extérieure du tuyau (2),
- une section de guidage formée sur la pièce de soupape (1 ; 40 ; 41 ; 50), et
- un guidage attribué à la pièce de soupape (1 ; 40 ; 41 ; 50), lequel coopère avec la section de guidage lors d'un mouvement relatif entre le guidage et la pièce de soupape (1 ; 40 ; 41 ; 50) dans le sens longitudinal du tuyau (2), de sorte que lors du mouvement relatif, l'élément de serrage (10 ; 40a, 40b ; 41a, 41b ; 51, 52, 53) est déplacé, modifiant ainsi la pression sur la surface extérieure du tuyau (2) et donc également l'écoulement à travers le canal à fluide (4),
**caractérisé en ce que** l'élément de serrage (10 ; 40a, 40b ; 41a, 41b ; 51, 52, 53) est agencé sur la pièce de soupape (1 ; 40 ; 41 ; 50) au moyen d'une fixation conçue de manière à permettre un déplacement répété très rapide de l'élément de serrage (10 ; 40a, 40b ; 41a, 41b ; 51, 52, 53), avec une fréquence entre 10 Hz et 500 Hz, lors du mouvement relatif pour l'ouverture et la fermeture au moins partielle du canal à fluide dans le tuyau (2).

2. Dispositif de soupape selon la revendication 1, **caractérisé en ce que** la pièce de soupape (1 ; 40 ; 41 ; 50) est formée avec un bras en saillie déplaçable et réversible, sur lequel est agencé l'élément de serrage (10 ; 40a, 40b ; 41a, 41b ; 51, 52, 53).

3. Dispositif de soupape selon la revendication 2, **caractérisé en ce que** le bras en saillie s'étend le long du tuyau (2) et forme un angle aigu au choix avec le sens longitudinal du tuyau (2).

4. Dispositif de soupape selon l'une au moins des revendications précédentes, **caractérisé en ce que** la section de guidage est formée sur l'élément de serrage (10 ; 40a, 40b ; 41a, 41b ; 51, 52, 53).

5. Dispositif de soupape selon l'une au moins des revendications précédentes, **caractérisé en ce que** le guidage est formée sur une section de paroi.

6. Dispositif de soupape selon l'une au moins des revendications précédentes, **caractérisé en ce que** la pièce de soupape est formée comme une micro-pièce de soupape.

7. Dispositif de soupape selon l'une au moins des revendications précédentes, **caractérisé en ce que** l'élément de serrage (40a, 40b ; 41a, 41b ; 51, 52, 53) est formé de manière à entourer le tuyau (2) au moins sur une moitié.

8. Dispositif de soupape selon l'une au moins des revendications précédentes, **caractérisé en ce que** l'élément de serrage est formé avec plusieurs éléments de serrage partiels (40a, 40b ; 41a, 41b ; 51, 52, 53) agencés autour du tuyau (2).

9. Dispositif de soupape selon la revendication 8, **caractérisé en ce qu'**au moins une partie de la pluralité d'élément de serrage partiels (51, 52, 53) est agencée de manière superposée dans au moins une position finale.

10. Dispositif de soupape selon l'une au moins des revendications précédentes, **caractérisé en ce que** la pièce de soupape (1 ; 40 ; 41 ; 50) est réalisée au moins partiellement comme une pièce moulée par injection.

11. Dispositif de soupape selon l'une au moins des revendications précédentes, **caractérisé en ce que** la pièce de soupape (1 ; 40 ; 41 ; 50) est agencée sur le tuyau (2).

12. Dispositif de soupape selon l'une au moins des revendications précédentes, **caractérisé en ce que** le canal à fluide s'étend à travers une section de compensation (14) compensant au moins partiellement le mouvement relatif entre le guidage et la pièce de soupape.

13. Système avec au moins un dispositif de soupape selon l'une au moins des revendications précédentes et un dispositif d'application de pression configuré pour mettre le fluide sous pression dans le canal à fluide.

14. Dispositif de distribution à plusieurs voies, avec plusieurs dispositifs de soupape selon l'une au moins des revendications 1 à 12.
